Europäisches Patentamt

European Patent Office

Office européen des brevets

Publication number: **0 347 026**

**A2**

# EUROPEAN PATENT APPLICATION

Application number: 89303597.2

Date of filing: 12.04.89

Int. Cl.⁴ **A61M 16/20 , A61M 1/00**

The title of the invention has been amended (Guidelines for Examination in the EPO, A-III, 7.3).

Priority: 19.04.88 CA 564529

Date of publication of application:
20.12.89 Bulletin 89/51

Designated Contracting States:
AT BE CH DE ES FR GB GR IT LI LU NL SE

Applicant: VENTECH HEALTHCARE CORPORATION INC.
Suite 1500, Canada Life Tower 736-6th Avenue S.A.
Calgary Alberta T2P 3T7(CA)

Inventor: Corris, Charles James
4 Silverwood Drive
Shenandoah, GA 30265(US)

Representative: Westwood, Edgar Bruce et al
STEVENS, HEWLETT & PERKINS 5, Quality Court Chancery Lane
London WC2A 1HZ(GB)

Valve for an endotracheal tube used for ventilation and aspiration.

An oxygenating catheter valve (10) for connection to an oxygen supply line (16) and to a suction line (14) for administration through a single catheter tube attachment (12). The control valve (10) is small and lightweight, having a simple spring loaded barrel (20) movable into and out of a cylinder (18). The barrel (20) has a cut away portion (50) shaped so that, in one position of the barrel (20), suction may be connected through the valve and, in another position of the barrel (20), oxygen is connected through the valve. Conveniently the spring loading is such that oxygen is automatically admitted in a rest position.

FIG.1.

# OXYGENATING CATHETER VALVE

## BACKGROUND OF THE INVENTION

### Field of the Invention

This invention is directed to a suction catheter system, and in particular to a two-way, three-port control valve.

### Background of the Invention

The medical application of such suction by use of suction catheters, particularly in relation to the lungs of a patient poses a number of problems.

In regard to accessing the lungs in normal fashion, by way of the mouth and throat, difficulties can arise particularly in the case of young children and babies, where the available throat opening permits passage at the maximum of a "#14 French" (about 5 mm diameter ) catheter, to be inserted.

United States Patent No. 4,193,406 issued March 18, 1980 to Jinotti discloses a catheter in which a valve spool has a first channel therethrough for aspiration and a second channel therethrough for oxygen. The spool is movable between a first position in which the first channel is connected between a catheter tube and a source of aspiration, the second channel venting to atmosphere, and second position in which the second channel is connected between a source of oxygen and the catheter tube and the first channel vents to atmosphere. The registration of the channels with the respective ports for oxygen supply, suction, and catheterization is critical and machining must be of a high degree of accuracy thus adding to manufacturing costs. Moreover, adequate sealing between fluid passages may provide problems.

U.S. Patent No. 4,705,073 to Beck discloses a catheterization valve having a chamber into which a catheter passage, an aspiration passage and an oxygenating passage all open. A slide is provided to selectively block and open the aspiration passage and the oxygenating passage before they open into the chamber. Spring biasing of the slide may call for accurate engineering and in some cases as in the case of the rectangular slide member of one of Beck's embodiments, two springs are suitably used for biasing the slide.

It would be desirable to provide a mechanically simple, inexpensive, simple to operate catheterization valve.

## SUMMARY OF THE INVENTION

According to the invention, an oxygenating catheter valve comprises a tubular valve housing having a catheter port adapted for connection to a catheter, an aspiration port adapted for connection to a source of suction and spaced from the catheter port, and an oxygenating port adapted for connection to a source of oxygen and spaced from the catheter port and spaced from the aspiration port; a valve spool axially movable in said valve housing and having a surface conforming with an inner surface of said valve housing, and having a channel in said surface adapted, in a first position of the spool to connect the oxygen port with the catheter port and, in a second position of the spool, to connect the aspiration port with the catheter port; fluid tight sealing means being provided for said channel between the spool and the housing; spring bias return means acting axially or the spool to bias it away from the second position towards said first position; and means for manually moving the spool to the second position against the spring bias return means.

## BRIEF DESCRIPTION OF THE DRAWINGS

Embodiments of the invention are described by way of example with reference to the drawings, in which:

Figure 1 is a perspective view of one valve according to the present invention;

Figure 2 is an exploded view of the valve of Figure 1;

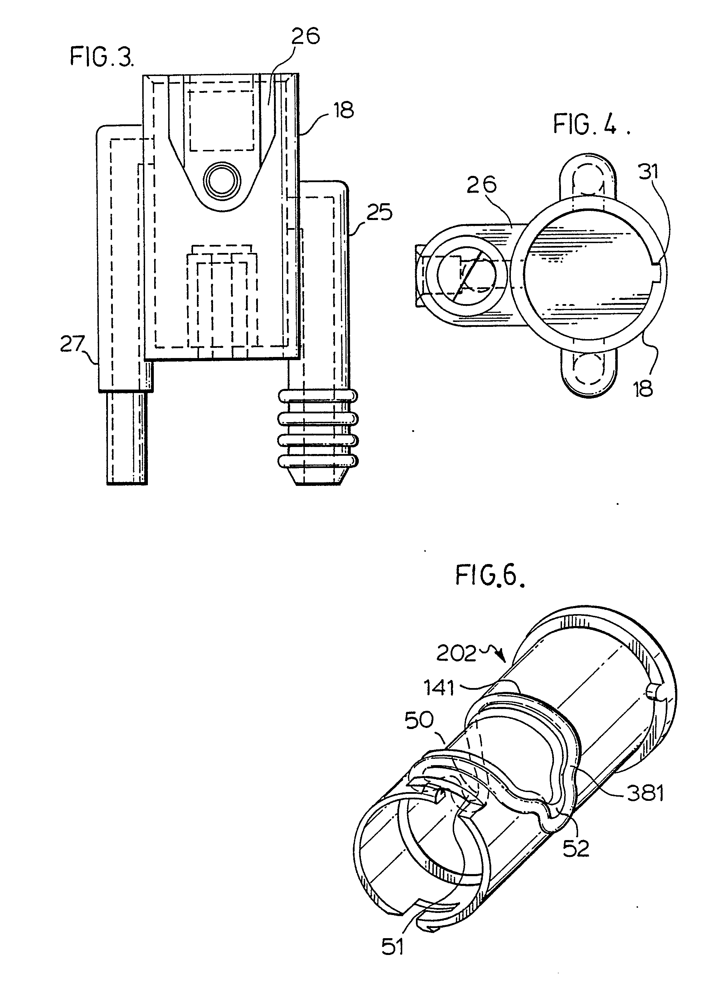

Figure 3 is a view of the valve body at right angles to that of Figure 2;

Figure 4 is an end view of the valve body;

Figure 5 is an exploded view similar to that of Figure 2 of a second embodiment of the valve; and

Figure 6 is a perspective view of a valve spool of a third embodiment.

## DESCRIPTION OF PREFERRED EMBODIMENTS

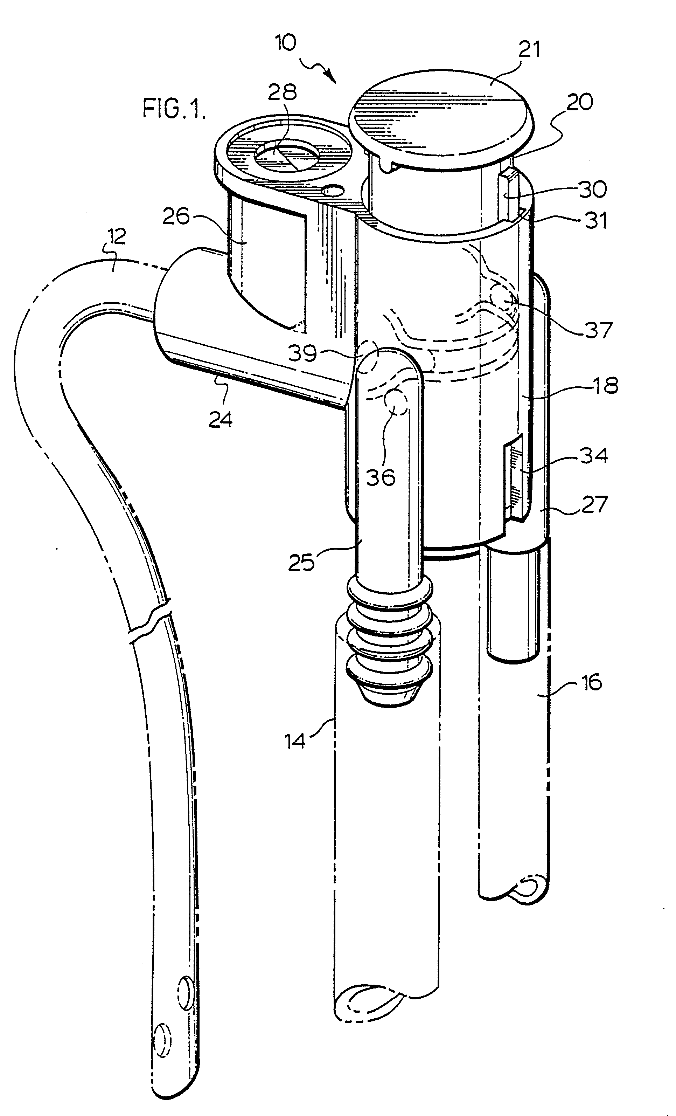

The drawings generally show a valve 10 having a catheter port 39, an aspiration port 36 and an oxygen port 37. These ports are respectively connectable to a catheter 12 through connector tubes 24, a suction line 14 through a connector 25, and an oxygen line 16 through connector 27, internal connections are shown in phantom in Figure 1. The connector of catheter 12 to connector 24 may be permanent as for example, by solvent welding.

The valve 10 comprises a tubular housing 18 having a valve spool 20 axially slidable therein. A biasing spring 22 acts between the valve spool 20 in a recess in the base thereof and an end wall of housing 18 to bias the spool partially out of the other end of the housing 18. The spool 20 is provided with an end member 21 through which finger pressure by an operator may depress the spool against spring bias into the housing 18. Means may be provided to inhibit rotation of the spool 20 in the housing 18, such as key 30 on spool 20 sliding in axially parallel keyway 31 on the housing 18. Of course. the slot may be provided in the spool and the key may be provided on the housing, if desired.

The valve spool 20 has a channel 50 recessed into its surface to provide a flow path at its periphery for oxygen or suction. The shape of the channel 50 and the disposition of the catheter port 39, the aspiration port 36 and the oxygenating port 37 of the housing 18 are such that, in a first position of the valve spool 20, the channel 50 connects the catheter port 39 and the oxygenating port 37, and, in a second position. the channel 50 connects the catheter port 39 and the aspiration port 36. Suitably, the first position in which oxygen may be delivered into the lungs of a patient is an at-rest position of the valve spool 20 as shown in phantom in Figure 1. From such a first position, the valve spool 20 may be depressed against bias of spring 22 into a second position in which channel 50 connects catheter port 39 with aspiration port 36. From this second position, the spool 20 will return to the first position on release of operator pressure on end member 21. Thus, the valve 10 automatically tends to return to a "safe" condition in which it delivers oxygen to the patient.

It is possible to provide a third neutral position for the valve spool such that the at-rest position is the third position in which neither aspiration nor oxygen is delivered to the patient. In this case, the valve spool 20 may be depressed by the operator from the third position to the first position for oxygenation and then to the second position for aspiration. Any convenient means may be provided to indicate to the operator, the appropriate operating position of the valve spool.

One suitable shape of channel 50 and configuration of the catheter port 39, the aspiration port 36 and the oxygen port 37 is as shown in the drawings. The catheter port 24 is located in housing 18 with the aspiration and oxygenating ports located to either side of it in each of axial and radial directions. The channel 50 is formed as an arcuate groove in cylindrical valve spool 20. The length of arc of channel 50 is such that at one end 52, the channel 50 registers with aspiration port 36 when the valve spool 20 is depressed into its second position. The width of the channel 50 at end 52 is such that, when the valve spool 20 moves away from the second position, channel 50 at end 52 moves out of registration with aspiration port 36.

Similarly, the length of arc of channel 50 is such that, at the other end 51, the channel 50 registers with oxygenating port 37 when the valve spool is in its first position. Again, the width of channel 50 at end 52 is such that, when the valve spool moves away from its first position, the channel 50 at end 51 moves out of registration with oxygenating port 37.

The mid portion of channel 50 is sufficiently wide that it registers with the catheter port 39 in both the first and second positions of valve spool 20.

While the shape of channel 50 and disposition of catheter, oxygenating and aspiration ports have been described in the above manner, it is clear that many other shapes and dispositions may be available. For example, channel 50 may be a diamond or elliptical or other channel having a central island. Thus, the catheter port would only communicate the channel at either of the first or second position, but not therebetween. Moreover. the channel 50 may be shaped to conform with configurations of the ports in which for example, the aspiration and oxygenating ports are aligned in an axial direction or are assymmetrically arranged about the catheter port.

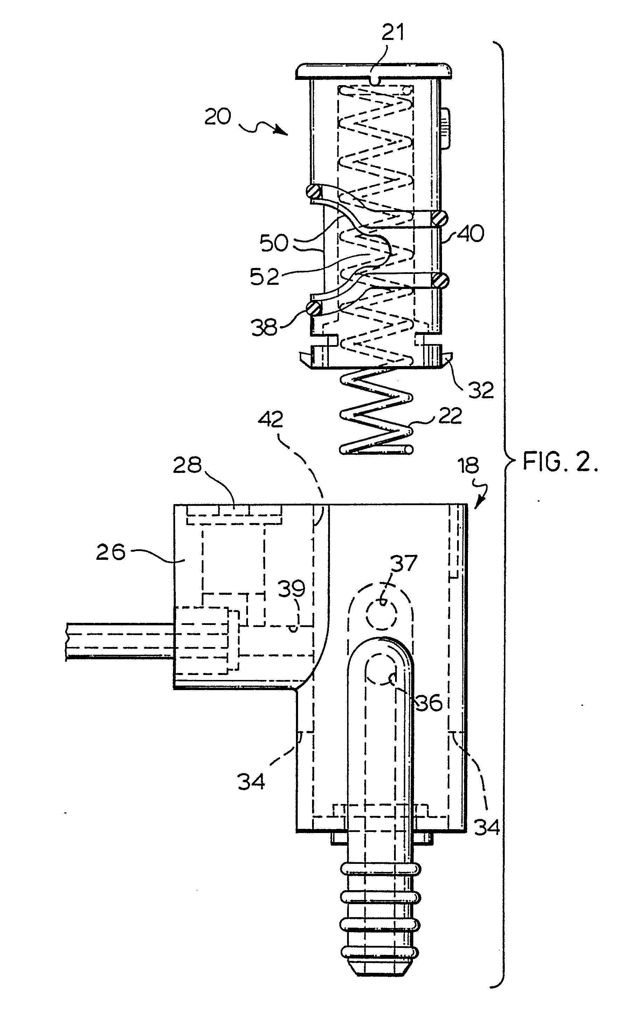

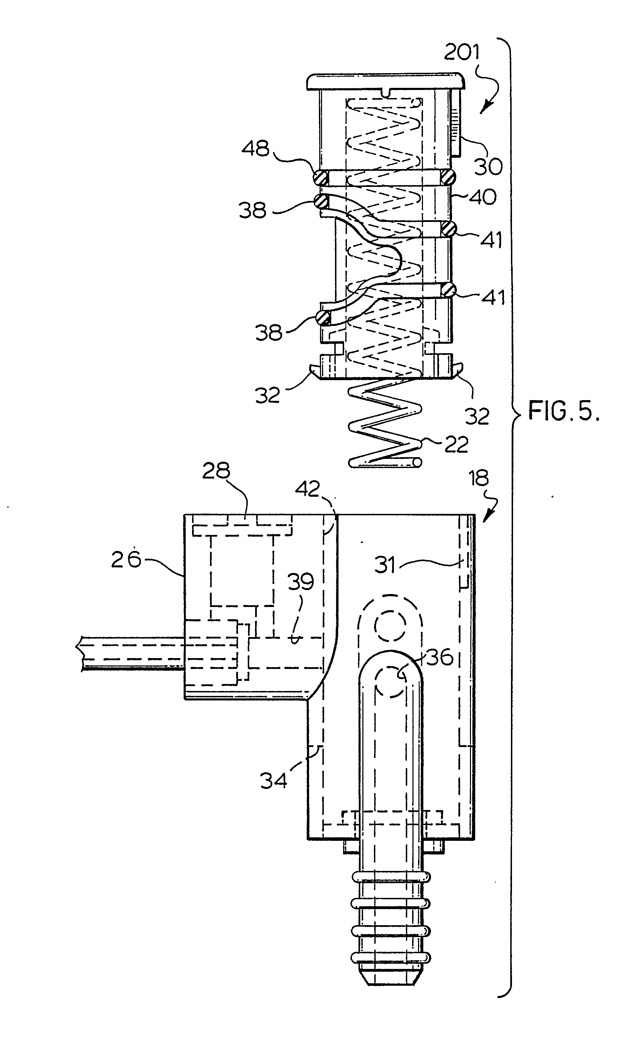

The channel 50 may be provided with sealing means against either leakage of suction and aspirated liquid or leakage of oxygen between the valve housing 18 and valve spool 20. The sealing means may be, for example, a pair of sealing O-rings 38 encircling a barrel portion 40 of spool 20. The O-rings 38 may be recessed within peripheral grooves 41 as shown in Figure 2 or 5 in the barrel portion 40 such that the radially outer surfaces of O-rings 38 protrude above the surface of barrel portion 40 into sealing relation with the inner wall surface of bore 42 of housing 18.

In the case of the Figure 5, a further O-ring 48 is additionally provided.

When O-ring 48 is not present and spool 20 is in its second position for aspiration, the oxygenating port is above upper sealing ring 38. If the fit of barrel 42 of housing 18 is sufficiently loose, there will be leakage of oxygen from the valve between the spool 20 and the housing 18. This leaked oxygen will emerge just below end member 21. This may be of advantage to maintain oxygen pressure from any sudden changes, but if it is desired to avoid this leakage, then O-ring 48 is provided.

Alternatively, a single seal 381 may be provided in a continuous groove 141 which does not extend about the barrel 40 of valve spool 20 but rather about the channel 50 (see Figure 6). When this single sealing ring 381 is used, it may be supplemented, if desired, by an additional ring such as ring 48 shown in Figure 5.

Spool 20 may have resilient tabs 32 which project radially outwardly from the body of the spool (See Figures 2 and 5). In this case, the valve housing 18 may have slots 34 to engage the tabs 32 to engage the valve spool 20 with the housing 18. The length of slots 34 may correspond with the distance of travel of the valve spool 20 between the first and second positions.

One embodiment, the slots 34 penetrate the housing 18, to provide ventilation to the housing.

When slots 34 communicate the interior of housing 18 to the atmosphere or the interior of the housing is otherwise vented to atmosphere, the valve spool 20 and the location of the aspiration port 36 may be such that, in the first position of spool 20 for oxygen delivery to the catheter 12, the suction line 14 vents to atmosphere. This may help to avoid build up of pressure which may be released sharply on communication of suction to the lungs of the patient.

A split seal flexible diaphragm 28 located in housing upper portion 26 may provide access to the interior of housing 18 and communication with the catheter 12. A lavage needle or tube may be introduced through the split diaphragm 28. The split diaphragm may also release undue internal pressure against excess oxygen pressure, or to admit atmosphere under undue suction being applied to the valve.

The use of a single O-ring seal embodiment as illustrated in Figure 7 makes it possible to provide venting of the lower portion of housing 18 to atmosphere by venting means running in an axially parallel direction between the blind ends 51 of channel 50. In this case, slots 34 may be blind slots, not penetrating the housing 20, thereby making the locking tabs 32 inaccessible.

It will be understood that the details of the subject valve can be modified, within the scope of the attached claims.

## Claims

1. An oxygenating catheter valve (10) comprising:
a tubular valve housing (18) having a catheter port (39) adapted for connection to a catheter (12), an aspiration port (36) adapted for connection to a source of suction and spaced from the catheter port (39), and an oxygenating port (37) adapted for connection to a source of oxygen and spaced from the catheter port (39) and spaced from the aspiration port (36);
a valve spool (20) axially movable in said valve housing (18) and having a surface conforming with an inner surface of said valve housing, and having a channel in said surface adapted, in a first position of the spool to connect the oxygen port (37) with the catheter port (39) and, in a second position of the spool, to connect the aspiration port (36) with the catheter port (39);
fluid tight sealing means (38, 381) being provided for said channel (50) between the spool (20) and the housing (18);
spring bias return means (22) acting axially on the spool (20) to bias it away from the second position towards said first position; and
means (21) for manually moving the spool (20) to the second position against the spring bias return means (22).

2. The valve as set forth in claim 1, including pressure relief means (28), adapted to vent the catheter to atmosphere at a predetermined overpressure.

3. The valve as set forth in claim 2, in which the pressure relief means (28) comprises a diaphragm valve.

4. The valve as set forth in claim 3, in which the diaphragm valve (28) is adapted for the introduction of a needle or tube.

5. The valve as set forth in claim 1, including a leakage path (34) to vent the suction line to atmosphere when said spool is in said first position.

6. The valve as set forth in claim 1, including leakage path to vent the oxygenating line to atmosphere when said spool is in said second position.

7. The valve as set forth in claim 1, said housing having a cylindrical internal wall defining a cylindrical bore therein; said valve spool means having a cylindrical body portion, sealing O-rings (48) adapted to seal between said housing and said spool being located in grooves about the cylindrical body portion above and below said channel.

8. The valve as set forth in claim 1, said valve spool means having an endless groove (50) in the surface thereof surrounding said channel and a sealing O-ring (381) adapted to seal between said housing and said spool being located in said groove.

FIG.1.

EP 0 347 026 A2

FIG. 2.

FIG.3.

26

18

25

27

FIG. 4.

26

31

18

FIG.6.

202

141

50

381

52

51

FIG.5.